# EUROPEAN PATENT APPLICATION

(11) **EP 2 670 131 A2**
(43) Date of publication of application: **04.12.2013**
(21) Application number: 13169380.6
(22) Date of filing: 27.05.2013
(51) Int. Cl.: H04N 5/367

(54) **Videoscope and image correcting method thereof**

(30) Priority: 28.05.2012 JP 2012120644
(71) Applicant: Fujifilm Corporation, Minato-ku Tokyo (JP)
(72) Inventor: Uchihara, Masanobu, Ashigarakami-gun Kanagawa (JP)
(74) Representative: Klunker . Schmitt-Nilson . Hirsch

(57) **Abstract**

A videoscope includes: an imaging device having at least one monitoring subject pixel each of which produces, frame by frame, a normal detection value or an abnormal detection value, normal pixels and at least one defective pixel; a memory which is stored with a position address of each of the at least one monitoring subject pixel; and an image processing unit which performs image processing on image signals produced by the imaging device so as to judge, in each frame, based on information stored in the memory, whether each of the at least one monitoring subject pixel has produced a normal detection value or an abnormal detection value and to correct the detection value of each of the at least one monitoring subject pixel using detection values of nearby normal pixels only in frames in which an abnormal detection value is produced.

## Description

### FIELD OF THE INVENTION

The present invention relates to a videoscope (electronic endoscope) in which a scope tip portion incorporates an imaging device. In particular, the invention relates to a videoscope and its image correcting method which can suppress a flicker of an image that is taken in a dark state.

### BACKGROUND OF THE INVENTION

Recent image sensors (imaging devices) have come to be provided with a very large number of pixels (photodiodes), and hence it is very difficult to manufacture all pixels on a photodetecting surface without a defect. It is a current situation that an imaging device needs to be used in a product even if it has defective pixels as long as they are not very large in number.

However, when a moving image of a subject is taken by an imaging device having defective pixels, fixed-pattern noise due to the defective pixels is superimposed on each frame of the moving image, as a result of which the image quality of the moving image is lowered to a large extent.

In view of the above, as described in JP-A-2008-148115 and JP-A-2006-26234, now it is a common procedure to interpolate (estimate) an image signal at each defective pixel using image signals of normal pixels around it and generate a subject image of each frame using estimated values.

Fixed-pattern noise due to defective pixels can be eliminated properly by interpolating an image signal at each defective pixel using image signals of normal pixels around it. However, even if image signals at defective pixels are corrected according to the method disclosed in JP-A-2008-148115 or JP-A-2006-26234, a flicker may occur in a moving image on the screen in, for example, a dark environment.

When the inside of a body cavity is observedby an endoscope, a bright portion and a dark portion occur in an image taken depending on a scope insertion state. In particular, when a moving image is taken in a dark environment, a flicker may be seen on the screen due to a reason other than defective pixels.

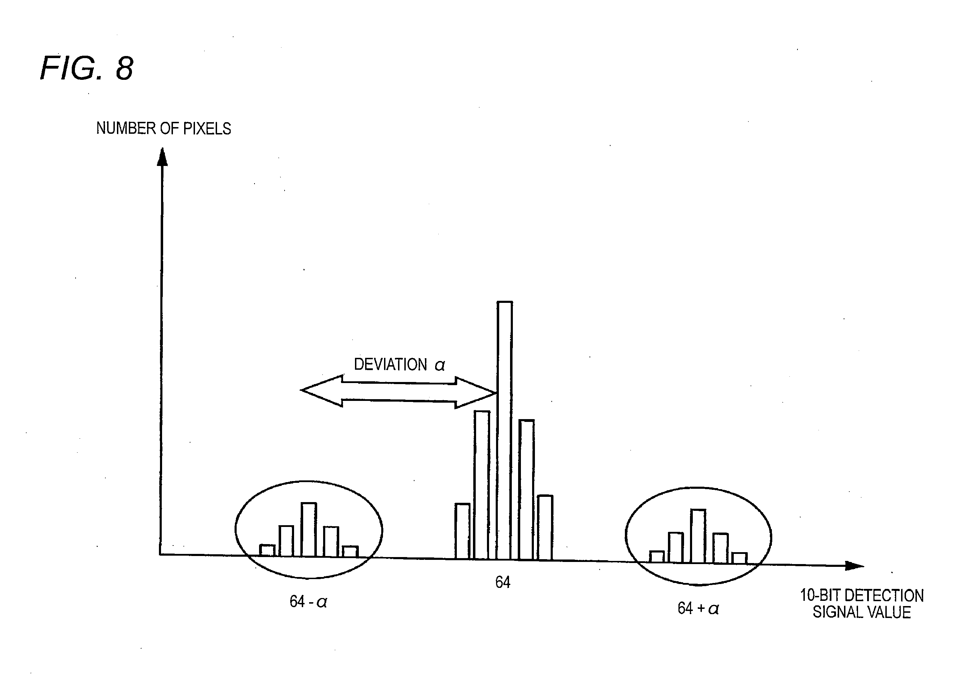

Such a flicker on the screen is not caused by defective pixels each of which produces an abnormal output value every frame but by pixels each of which produces a normal output value or a value that deviates from a normal output value depending on the frame, that is, discretely produces abnormal output values. Fig. 8 is a graph showing black level detection values. Although deviations of this kind occur in the same manner in a dark image and a bright image, they are conspicuous (recognized as noise) in a dark image because of low signal levels whereas they are not conspicuous in a bright image because of high signal levels.

When a pitch-dark scene is taken only by normal pixels of an imaging device, the detection value of each pixel is not "0" but a certain value. For example, assume that an imaging device having 10-bit (1,024) gradation levels employs a pixel value "64" as a black level judging threshold value.

Although many normal pixels constantly produce detection values that are close to the pixel value "64" in attempts to shoot a pitch-dark scene, there are pixels each of which produces detection values that fluctuate like "50," "64," "80," ··· from one frame to another in each attempt to shoot a pitch-dark scene. That is, there are pixels whose black level detection values fluctuate in a range of 64 ± α. When shooting is performed in a dark environment, this fluctuation causes flicker noise in a moving image on the screen.

This phenomenon is considered due to combinations of pixels and correlated double sampling processing circuits, amplifiers, and A/D converters. In recent imaging devices, to increase the reading rate, these amplifiers etc. are provided for each pixel row (or column) to enable parallel processing.

However, since it is difficult to manufacture completely the same processing circuits for the respective pixel rows (or columns), fluctuations of α as mentioned above would occur depending on the combinations of the pixel rows (or columns) and the amplifiers etc. There are imaging devices in which the combinations of the pixel rows (or columns) and the amplifiers etc. are switched at a prescribed cycle because fixed-pattern noise may occur if the combinations are fixed. This is also a cause of a flicker which occurs in a moving image in a dark shooting environment.

### SUMMARY OF THE INVENTION

An object of the present invention is to a videoscope and its image correcting method which suppress flicker noise on the screen even when a moving image is taken in a dark shooting environment.

The invention provides a videoscope which has an imaging device having, in addition to normal pixels and at least one defective pixel, at least one monitoring subject pixel each of which produces a normal detection value or an abnormal detection value depending on the frame (frame by frame), a memory stored with a position address of each of the at least one monitoring subject pixel, and an image processing unit as well as its image correcting method, **characterized in that**:
in performing image processing on image signals produced by the imaging device, whether each of the at least one monitoring subject pixel has produced a normal detection value or an abnormal detection value is judged in each frame on the basis of information stored in the memory, and the detection value of each of the at least onemonitoring subj ect pixel is corrected using detection values of nearby normal pixels only in frames in which it produces an abnormal detection value.

The invention makes it possible to suppress flicker noise in a moving image displayed on a monitor even when it is taken in a dark shooting environment.

### BRIEF DESCRIPTION OF THE DRAWINGS

Fig. 1 shows the configuration of the entire system of a videoscope according to an embodiment of the present invention.
Fig. 2 is a front view of a tip surface of a tip portion of the videoscope system shown in Fig. 1.
Fig. 3 is a vertical sectional view of the tip portion of the videoscope system shown in Fig. 1.
Fig. 4 is a block diagram showing the configuration of a control system of the videoscope system shown in Fig. 1.
Fig. 5 is a flowchart showing the procedure of an image correction process which is executed by a processor unit shown in Fig. 4.
Fig. 6 is a flowchart showing a detailed procedure of a flicker correction step shown in Fig. 5.
Fig. 7 is a schematic diagram of the surface of a solid-state imaging device having a color filter whose color segments are Bayer-arranged.
Fig. 8 is a graph illustrating presence of pixels whose black level detection values fluctuate among normal pixels of an imaging device.

### [Description of symbols]

1: Monitoring subject pixel
2, 3, 4, 5, 6, 7, 8, 9: Nearby pixel of the same color
10: Videoscope system
12: Scope
14: Processor unit
16: Light source unit
26: Tip portion
38: Monitor
40: Observation window
42: Illumination window
50: Objective optical system
54: Imaging chip
56: Prism
58: Imaging device (image sensor)
62: Board
68: Cable
80, 82, 104: CPU
81: Memory (EEPROM)
84: ROM
86: DSP

### DETAILED DESCRIPTION OF THE INVENTION

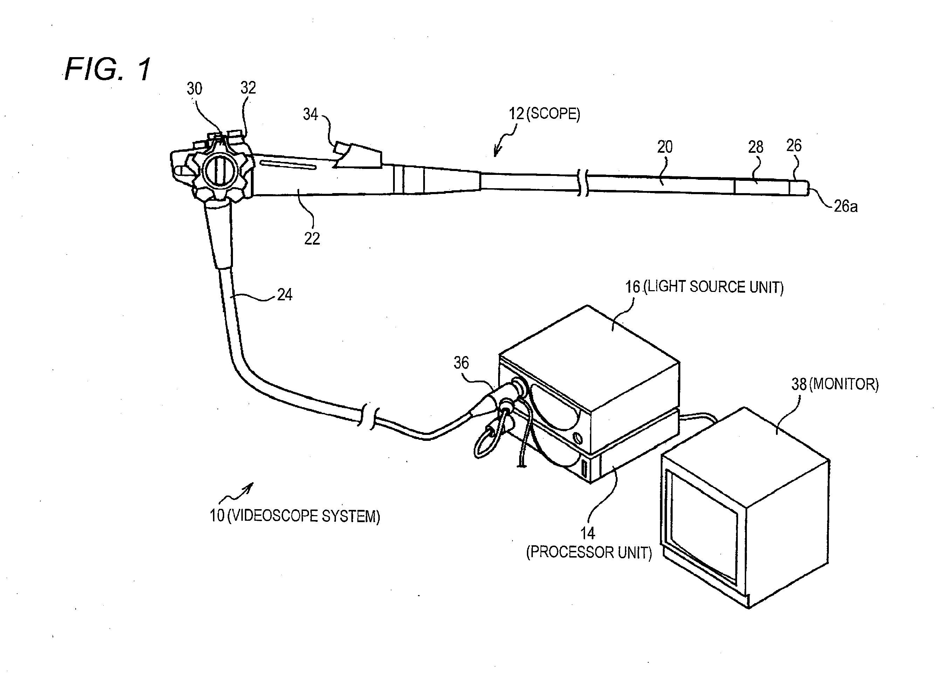

An embodiment of the present invention will be hereinafter described with reference to the drawings. Fig. 1 shows the configuration of the entire system of a videoscope according to the embodiment of the invention. The videoscope system 10 according to the embodiment is composed of a scope 12 and a processor unit 14 and a light source unit 16 which constitute a main apparatus. The scope 12 is equipped with a flexible insertion portion 20 to be inserted into the body cavity of a patient (subject person), a manipulation unit 22 which is connected to a proximal portion of the insertion portion 20, and a universal cord 24 which is connected to the processor unit 14 and the light source unit 16.

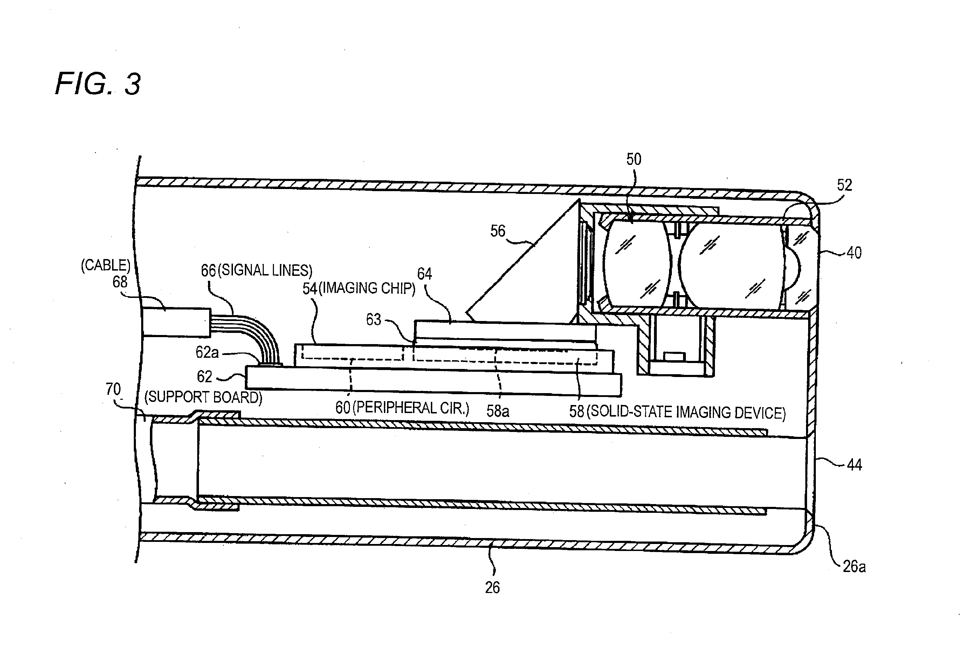

A tip portion 26 is provided at the tip of the insertion portion 20, and incorporates an imaging chip (imaging device) 54 (see Fig. 3) for shooting the inside of a body cavity. A curving portion 28 which is a connection of plural curving pieces is provided on the proximal side of the tip portion 26. When an angling knob of the manipulation unit 22 is manipulated, a wire that is inserted in the insertion portion 20 is pushed or pulled, whereby the curved portion 28 is curved in any direction. In this manner, the tip portion 26 can be directed to a desired direction in a body cavity.

A connector 36 is provided at one end of the universal cord 24. Being of a composite type, the connector 36 is connected not only to the processor unit 14 but also the light source unit 16.

The processor unit 14 supplies power to the scope 12 and controls driving of the imaging chip 54 via a cable 68 (see Fig. 3) which is inserted through the universal cord 24. Furthermore, the processor unit 14 receives image signals that are transmitted from the imaging chip 54 by the cable 68, and converts them into signal data by performing various kinds of signal processing on it.

The image data produced by the processor unit 14 is displayed as an endoscope image (observation image) on a monitor 38 which is cable-connected to the processor unit 14. Also electrically connected to the light source unit 16 via the connector 26, the processor unit 14 controls the operations of the videoscope system 10 including the light source unit 16 in a unified manner.

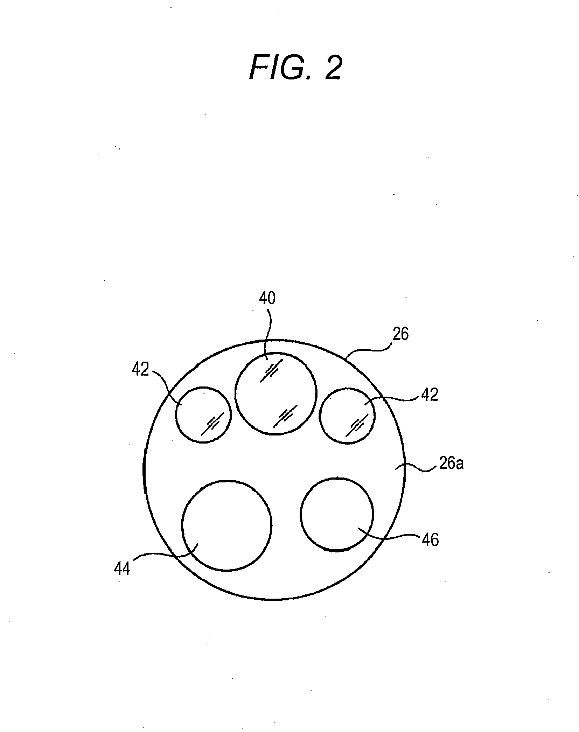

Fig. 2 is a front view of a tip surface 26a of the tip portion 26 of the scope 12. As shown in Fig. 2, the tip surface 26a of the tip portion 26 is provided with an observation window 40, two illumination windows 42, a forceps outlet 44, and an air/water supply nozzle 46.

In Fig. 2, the observation window 40 is located on the vertical center line and is deviated upward from the center. The two illumination windows 42 are located symmetrically with respect to the vertical center line on which the observation window 40 is located, and serve to apply illumination light beams coming from the light source unit 16 to a part to be observed in a body cavity.

The forceps outlet 44 is connected to a forceps pipe 70 (see Fig. 3) which extends through the insertion portion 20, and thereby communicates with a forceps inlet 34 (see Fig. 1) which is formed in the manipulation unit 22. One of various treatment tools in each of which an injector needle, a high-frequency scalpel, or the like is attached to the tip is inserted through the forceps inlet 34, and a tip portion of the treatment tool is projected into a body cavity through the forceps outlet 44.

The air/water supply nozzle 46 jets out, toward the observation window 40 or into a body cavity, cleaning liquid or air that is supplied from an air/water supply device which is provided in the light source unit 16 when an air/water supply button 32 (see Fig. 1) of the manipulation unit 22 is manipulated.

Fig. 3 is a vertical sectional view of the tip portion 26 of the scope 12. As shown in Fig. 3, a lens barrel 52 which holds an objective optical system 50 for taking in light coming from a part to be observed in a body cavity is disposed behind the observation window 40. The lens barrel 52 is attached so that the optical axis of the objective optical system 50 is made parallel with the center axis of the insertion portion 20. A prism 56 for guiding, toward the imaging chip 54, light coming from a part to be observed via the objective optical system 50 by bending it by approximately 90° is connected to the rear end of the lens barrel 52.

The imaging chip 54 is a monolithic semiconductor (sensor chip) in which a solid-state imaging device 58 and peripheral circuits 60 for driving the solid-state imaging device 58 and performing signal input/output processing are integrated together. The imaging chip 54 is mounted on a support board 62.

An imaging surface (photodetecting surface) 58a of the solid-state imaging device 58 is opposed to the light exit surface of the prism 56. A cover glass 64 which is shaped like a rectangular plate is attached to the imaging surface 58a via a spacer 63 which is shaped like a rectangular frame. The imaging chip 54, the spacer 63, and the cover glass 64 are assembled together by adhesive, whereby the imaging surface 58a is protected from entrance of dust etc.

Plural input/output terminals 62a are formed on a rear end portion of the support board 62 which extends toward the rear end of the insertion portion 20, so as to be arranged in the width direction of the support board 62. Signal lines 66 for exchange of various signals with the processor unit 14 via the universal cord 24 are joined to the input/output terminals 62a. The input/output terminals 62a are electrically connected to the peripheral circuits 60 of the imaging chip 54 via interconnections, bonding pads, etc. (not shown) formed on the support board 62.

The signal lines 66 are inserted together through a flexible pipe of the cable 68. The cable 68 is inserted through the insertion portion 20, the manipulation unit 22, and the universal cord 24 and connected to the connector 36.

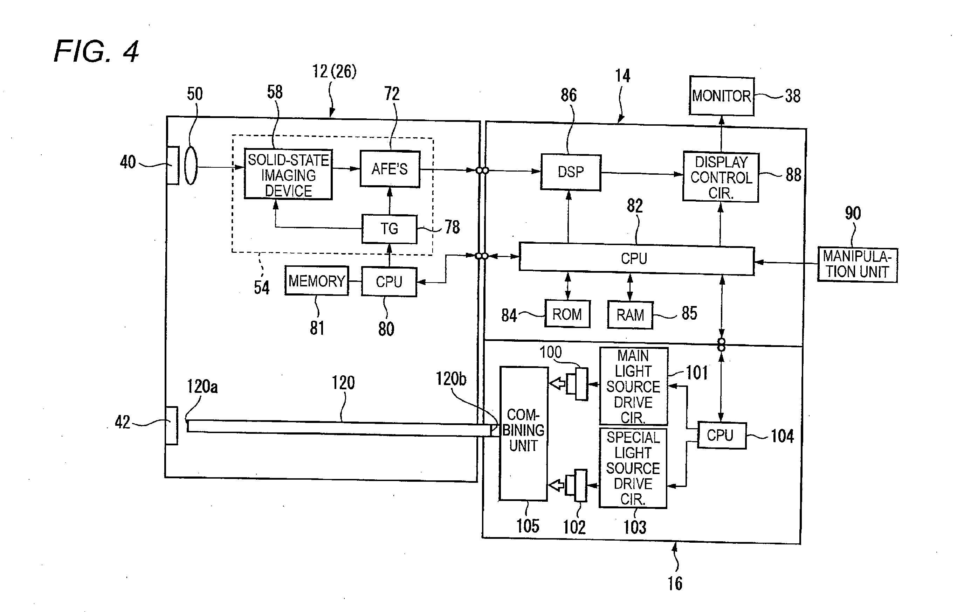

Although not shown in Figs. 2 and 3, an illumination unit is disposed behind each illumination window 42. The illumination unit is provided with an emission end 120a of a lightguide 120 (see Fig. 4) for guiding illumination light that is supplied from the light source unit 16, and the emission end 120a is opposed to the illumination window 42. Like the cable 68, the lightguide 120 is inserted through the insertion portion 20, the manipulation unit 22, and the universal cord 24, and its incidence end is connected to the connector 36.

Fig. 4 is a block diagram of a control system of the videoscope system 10. As shown in Fig. 4, the tip portion 26 of the scope 12 is provided with the solid-state imaging device 58, analog signal processing circuits (AFEs: analog front ends) 72, a timing generator (TG) 78, and a CPU 80. The AFEs 72 and the TG 78 correspond to the peripheral circuits 60 shown in Fig. 3.

A memory 81 such as an EEPROM is connected to the CPU 80. The memory 81 is stored with data specific to the scope 12 and data specific to the solid-state imaging device 58, such as position data of defective pixels of the solid-state imaging device 58.

Controlled by the CPU 80, the TG 78 generates drive pulses (vertical and horizontal scanning pulses, reset pulses, etc.) for the solid-state imaging device 58 and sync pulses for the AFEs 72. The solid-state imaging device 58 is driven by the drive pulses supplied from the TG 78, and performs photoelectric conversion on an optical image formed on the imaging surface 58a by the objective optical system 50 and outputs resulting image signals.

A large number of pixels are arranged on the imaging surface 58a of the solid-state imaging device 58 in matrix form, and the pixels are provided with respective photosensors (photoelectric conversion elements). Light that is incident on the imaging surface 58a of the solid-state imaging device 58 produces charges that are accumulated in the photosensors of the pixels, respectively. Amounts of signal charges accumulated in the photosensors of the pixels are read out sequentially as pixel signals by scanning in the vertical and horizontal directions which is performed by a vertical scanning circuit and a horizontal scanning circuit (neither of which is shown), and are output at a prescribed frame rate.

The solid-state imaging device 58 is a single-chip color imaging type solid-state imaging device which is equipped with a color filter having color segments of plural colors (e.g., Bayer-arranged primary color filter).

The configuration of a signal reading circuit for reading out, as image signals, charges accumulated in the respective photosensors of the solid-state imaging device 58 is known (a common configuration such as a 3-transistor or 4-transistor configuration can be used), and hence is not described here.

Each of the AFEs 72 is composed of a correlated double sampling (CDS) circuit, an automatic gain control (AGC) circuit, and an A/D converter. The CDS circuit performs correlated double sampling on image signals that are output from the solid-state imaging device 58 and thereby eliminates reset noise and amplifier noise occurring therein. The AFEs 72 are provided for the respective pixel columns of the solid-state imaging device 58.

The AGC circuit amplifies the image signals as noise-eliminated by the CDS circuit at a gain that is specified by the CPU 80. The A/D converter converts the image signals as amplified by the AGC circuit into digital signals having a prescribed number of bits, and outputs the latter. The digital image signals that are output from the AFEs 72 are input to the processor unit 14 via the signal lines 66.

The processor unit 14 is composed of a CPU 82, a ROM 84, a RAM 85, an image processing circuit (DSP) 86, and a display control circuit 88.

The CPU 82 controls the individual units of the processor unit 14 and also controls the entire videoscope system 10 in a unified manner. The ROM 84 is stored in advance with various programs, control data, etc. for controlling the operations of the processor unit 14. The RAM 85 is temporarily stored with a program to be executed by the CPU 82, related data, etc.

The DSP 86 generates image data by performing flicker correction processing, color interpolation, color separation, color balance adjustment, gamma correction, image emphasis processing, etc. on image signals that are input from the AFEs 72, under the control of the CPU 82.

The image data that is output from the DSP 86 is input to the display control circuit 88, which converts the received image data into data having such a signal format as to be suitable for the monitor 38 and displays a corresponding image on the screen of the monitor 38.

Amanipulation unit 90 of the processor unit 14 is provided with a mode switching button (s) for selecting one of or switching between operation modes of the solid-state imaging device 58 and various buttons for receiving inputs of other kinds of user instructions.

The light source unit 16 is composed of a main light source 100, a main light source drive circuit 101, a special light source 102, a special light source drive circuit 103, a CPU 104, and a combining unit 105. Communicating with the CPU 82 of the processor unit 14, the CPU 104 controls the main light source drive circuit 101 and the special light source drive circuit 103.

Themain light source 100 emits white light and the special light source 102 emits special light in a narrow band which is centered by 420 nm, for example. The white light or the special light shines on the incidence end 120b of the lightguide 120 via the combining unit 105.

The inside of a body cavity is observed in the following manner using the above-configured videoscope system 10. After the scope 12, the processor unit 14, the light source unit 16, and the monitor 38 are powered on, the insertion portion 20 of the scope 12 is inserted into the body cavity and a moving image of the inside of the body cavity which is taken by the solid-state imaging device 58 and displayed on the monitor 38 is observed while the inside of the body is illuminated by illumination light guided from the light source unit 16.

In generating an image to be displayed on the monitor 38, the DSP 86 takes in image signals (raw signals) that are output from the imaging chip 54 and performs flicker correction processing. Then, the DSP 86 performs known various kinds of image processing such as synchronization processing (demosaicking), gamma correction, and RGB/YC conversion on flicker-corrected image signals corresponding to the respective pixel positions, and thereby generates an image to be displayed on the monitor 38.

Image correction processing (flicker correction processing) according to the embodiment will be described below.

The pixels of the solid-state imaging device 58 are divided into normal pixels and defective pixels. Image signals of the defective pixels are corrected by a known method. That is, communicating with the CPU 80 of the scope 12, the CPU 82 of the processor unit 14 acquires the defective pixel position information (stored in the memory 81) of the solid-state imaging device 58 incorporated in the scope 12 and passes it to the DSP 86. Based on this information, the DSP 86 calculates an image signal at each defective pixel position using image signals of nearby pixels through pixel interpolation.

As described above with reference to Fig. 8, not all normal pixels suffer fluctuation of detection values. Pixels that suffer fluctuation of detection values can be determined by an advance test. These pixels are made monitoring subject pixels, and their pixel positions (pixel addresses) are written to the memory 81 like those of the defective pixels. Communicating with the CPU 80, the CPU 82 acquires the pixel position information of the monitoring subject pixels and passes it to the DSP 86. The DSP 86 performs flicker correction processing in the following manner.

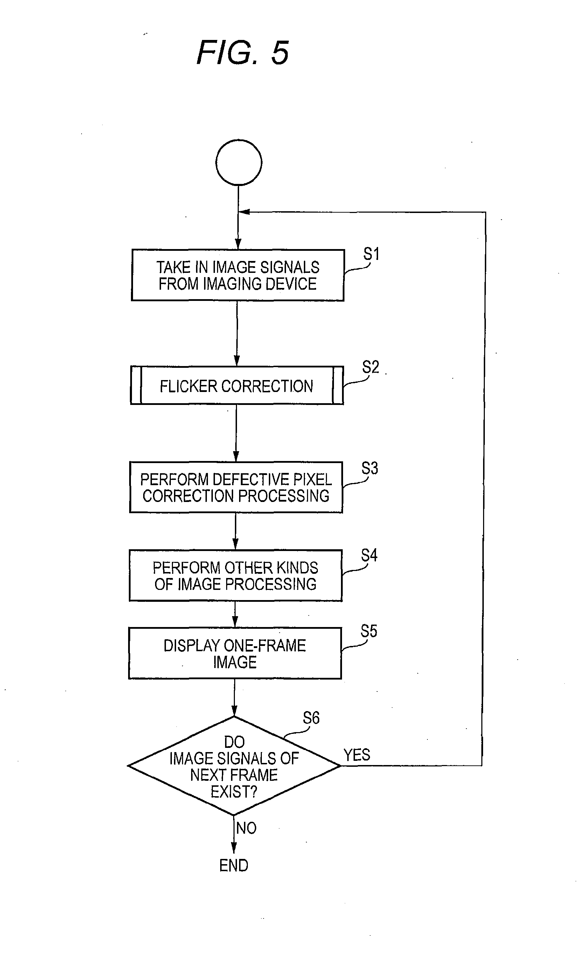

Fig. 5 is a flowchart showing the procedure of an image correction process which is executed by the CPU 82 of the processor unit 14 using the DSP 86 etc. that are subordinate to it. First, at step S1, image signals that are output from the solid-state imaging device 58 are taken in.

At step S2, a flicker correction (described later in detail) is performed is performed. At step S3, known defective pixel correction processing is performed for each of defective pixels having pixel addresses that are read from the memory 81.

At step S4, other known kinds of image processing (γ correction, demosaicking,RGB/YC separation,etc.)are performed. At step S5, an image is displayed on the screen of the monitor 38 on the basis of 1-frame image data as subjected to those kinds of image processing. At step S6, it is judged whether image signals of the next frame exist or not. If no such image signals exist (i.e., the taking of a moving image has been finished), the process is finished.

If image signals of the next frame exist, the process returns from step S6 to step S1. Steps S1-S6 are executed repeatedly until the taking of a moving image is finished.

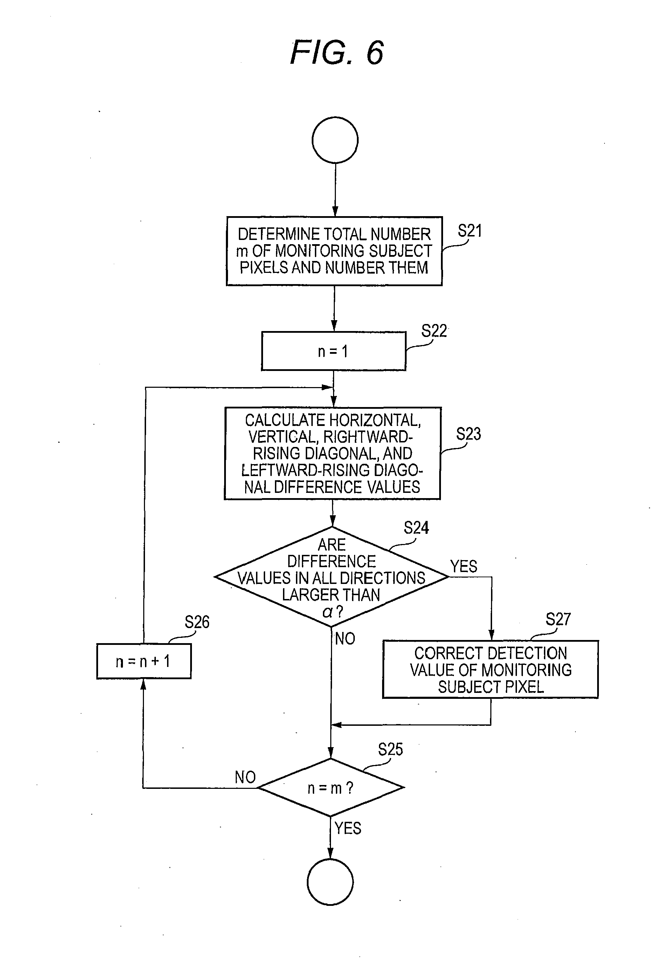

Fig. 6 is a flowchart showing a detailed procedure of step S2 (flicker correction) shown in Fig. 5. First, at step S21, a total number m of monitoring subject pixels is determined and the monitoring subject pixels are numbered. At step S22, a variable n is set to "1."

At step S23, for the first monitoring subject pixel (n =1), dif ferences between a detection value of itself and detection values of normal pixels of the same color that are not a monitoring subject pixel and are located on each of the vertical line, the horizontal line, and the rightward-rising 45° diagonal line, and the leftward-rising 45° diagonal line that cross the first monitoring subject pixel are calculated.

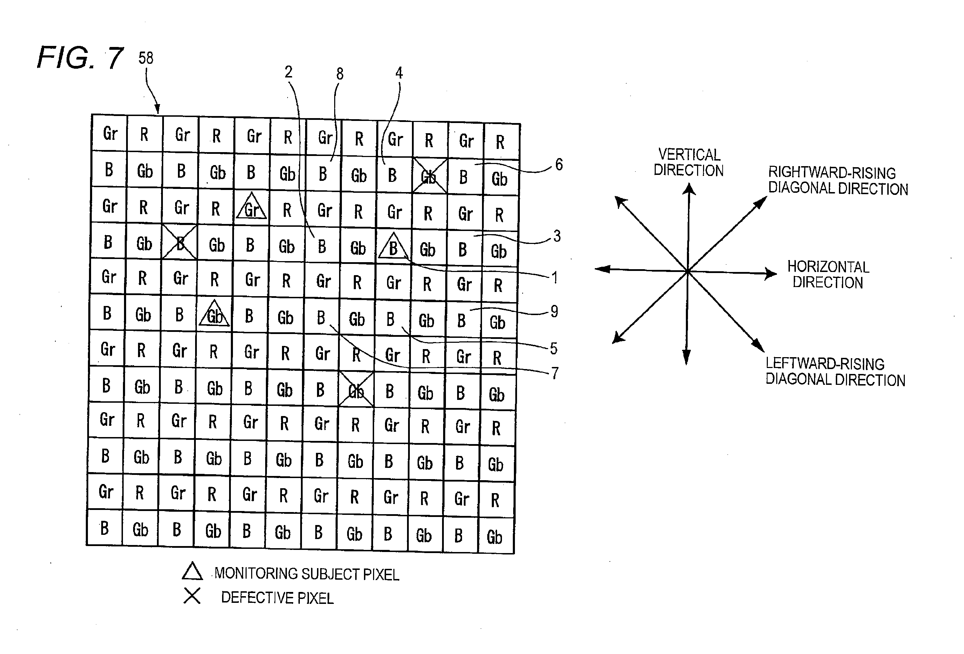

Fig. 7 is a schematic diagram of the surface of the solid-state imaging device 58. In this example, the pixels are arranged in square lattice form and color filter segments of the three primary colors R (red), G (green), and B (blue) are Bayer-arranged on the pixels. Filter segments of G in each row in which filter segments of B are also arranged are denoted by Gb, and filter segments of G in each row in which filter segments of R are also arranged are denoted by Gr. Although the filter segments of Gb and the filter segments of Gr have the same color, in the embodiment they are dealt with as if to have different colors. Naturally, they may be dealt with as having the same color.

In Fig. 7, defective pixels are indicated by marks "×," correction processing for which is performed at step S3 shown in Fig. 5. Monitoring subject pixels are indicated by marks "Δ," correction processing for which is performed according to the process shown in Fig. 6.

Now, attention is paid to a B pixel 1 which is a monitoring subject pixel. B pixels 2 and 3 exist on the left and right of the B pixel 1 in the same row with one pixel interposed in between. The average of the differences between a detection value of the B pixel 1 and detection values of the B pixels 2 and 3 is made a horizontal difference value H.

B pixels 4 and 5 exist over and under the B pixel 1 in the same column with one pixel interposed in between. The average of the differences between the detection value of the B pixel 1 and detection values of the B pixels 4 and 5 is made a vertical difference value V.

B pixels 6 and 7 exist on the top-right and bottom-left of the B pixel 1 on the rightward-rising 45° diagonal line with one pixel interposed in between. The average of the differences between the detection value of the B pixel 1 and detection values of the B pixels 6 and 7 is made a rightward-rising diagonal difference value R.

B pixels 8 and 9 exist on the top-left and bottom-right of the B pixel 1 on the leftward-rising 45° diagonal line with one pixel interposed in between. The average of the differences between the detection value of the B pixel 1 and detection values of the B pixels 8 and 9 is made a leftward-rising diagonal difference value L.

There may occur a case that one of the B pixels 2-9 is a defective pixel or a monitoring subject pixel. In such a case, a difference value H, V, R, or L is calculated without using a detection value of the defective pixel or the monitoring subj ect pixel. Rather than the average of two detection values, one detection value itself may be used as a difference value. For example, if both of the B pixels 2 and 3 are a defective pixel (s) or a monitoring subject pixel(s), detection values of B pixels that are more distant from the B pixel 1 than the B pixels 2 and 3 by two pixels.

After difference values H, V, R, and L in the four directions were calculated at step S23, at step S24 it is judges whether all of the four difference values H, V, R, and L are larger than a threshold value α.

If at least one of the four difference values H, V, R, and L is not larger than the threshold value α (S24: no), it is judged that the detection value of the first monitoring subject pixel (n = 1) is not a fluctuated value, that is, it is a normal value.

At step S25, it is judged whether n is equal to m, that is, whether detection values of all the monitoring subj ect pixels have been processed. If n is not equal to m, the process moves from step S25 to step S26, where n is incremented by 1. Then, the process returns to step S23. Step S23 and the following steps are executed for the second monitoring subject pixel (n = 2).

If it is judged at step S24 that all of the four difference values H, V, R, and L are larger than the threshold value α, it is judged that the detection value of the first monitoring subject pixel is a fluctuated value, that is, it is not a normal value. The process moves to step S27, where the detection value of the first monitoring subject pixel is replaced by a correction value. The process moves to step S25.

For example, the correction value used at step S27 is determined in the following manner. The smallest one of the four difference values H, V, R, and L is selected. For example, if the horizontal difference value H is smaller than each of the other difference values V, R, and L, the horizontal difference value H is selected. That is, in the example of Fig. 7, the B pixels 2 and 3 are selected for the B pixel 1 and the average of the detection values of the B pixels 2 and 3 is used as a detection value of the monitoring subject pixel 1. Alternatively, the detection value itself of the B pixel 2 or 3 is used as a detection value of the monitoring subject pixel 1.

The reason why a correction value for the monitoring subject pixel 1 is determined in the above-described manner is as follows. Assume that it is being attempted to take an image of narrow blood vessels (as a main subject image in a whole image taken). If an optical image of a single narrow blood vessel runs so as to pass the pixels 2, 1 and 3 in this order, it is highly probable that the difference value H is smaller than the other difference values V, R, and L, because the pixels 1, 2 and 3 are being used for taking an image of the same blood vessel. Since pixel 1 detection values of a dark image are unstable, an image of the narrow blood stream could be reproduced properly by replacing the detection value of the pixel 1 with the average of the detection values of the pixels 2 and 3.

In contrast, if a correction value for the monitoring subject pixel 1 is determined using the detection values of the pixels 3 and 4, the detection values of the pixels 2, 1 and 3 arranged in this order has a step, that is, a blood vessel image is disconnected.

The above-described processing is performed for all the monitoring subject pixels in each frame, as a result of which a flicker on the screen can be suppressed.

According to the above-described embodiment, even if there exist pixels each which produces unstable detection values to cause a flicker particularly in a dark environment, such detection values can be corrected properly and a high-quality monitor image can be obtained.

Monitoring subject pixels which are correction subject pixels of the embodiment are not detective pixels. A defective pixel is a pixel whose output value is deviated from a normal output value in every frame. Therefore, an output value of a defective pixel needs to be corrected using output values of nearby normal pixels in every frame. On the other hand, a monitoring subj ect pixel is a pixel which produces a normal output value or an abnormal output value depending on the frame (The monitoring subject pixel produces a normal output value for some of frames but produces an abnormal output value for the other of frames). In the case of a monitoring subject pixel, to avoid overcorrection, a detection value need not be interpolated using output values of nearby pixels in frames in which a normal detection value is produced. It suffices to interpolate a detection value only when an abnormal output value is produced.

Like defective pixels, normal pixels may turn into monitoring subject pixels with age. A measure to cope with this phenomenon is to inspect the scope 12 regularly and register, in the memory 81, addresses of pixels that have newly turned into monitoring subject pixels.

In the embodiment, at step S27 shown in Fig. 6, a correction value for the monitoring subject pixel is calculated using detection values of only a small number of (in the embodiment, two) pixels that are close to it. Alternatively, it is possible to determine a correction value for interpolation using detection values of nearby pixels of the same color that are located in the same row or column or on the same diagonal lines. However, using only pixels of the same color that are closest to the monitoring subject pixel to calculate a correction value is preferable because it does not cause a reduction in the resolution of an image taken.

As described above, the embodiment provides a videoscope which has an imaging device having, in addition to normal pixels and defective pixels, monitoring subject pixels each of which produces a normal detection value or an abnormal detection value depending on the frame, a memory stored with position addresses of the respective monitoring subject pixels, and an image processing unit as well as its image correcting method, **characterized in that**:
in performing image processing on image signals produced by the imaging device, whether each of the monitoring subject pixels has produced a normal detection value or an abnormal detection value is judged in each frame on the basis of information stored in the memory, and the detection value of each monitoring subj ect pixel is corrected using detection values of nearby normal pixels only in frames in which it produces an abnormal detection value.

The videoscope according to the embodiment is **characterized in that** the image processing unit deals with a monitoring subject pixel in the same manner as a normal pixel and does not correct its detection value if differences between its detection value and detection values of nearby normal pixels are within a prescribed range defined by a threshold value.

The video scope according to the embodiment is **characterized in that** the image processing unit corrects a detection value of a monitoring subject pixel if a horizontal difference value between the detection value of the monitoring subject pixel and a detection value or values of a nearby normal pixel or pixels that are located on the same horizontal line as the monitoring subject pixel, a vertical difference value between the detection value of the monitoring subject pixel and a detection value or values of a nearby normal pixel or pixels that are located on the same vertical line as the monitoring subject pixel, a rightward-rising diagonal difference value between the detection value of the monitoring subject pixel and a detection value or values of a nearby normal pixel or pixels that are located on the same rightward-rising diagonal line as the monitoring subject pixel, and a leftward-rising diagonal difference value between the detection value of the monitoring subject pixel and a detection value or values of a nearby normal pixel or pixels that are located on the same leftward-rising diagonal line as the monitoring subject pixel are all larger than the threshold value.

The videoscope according to the embodiment is **characterized in that** the image processing unit corrects the detection value of the monitoring subject pixel by replacing it with a detection value of a nearby normal pixel or an average of detection values of nearby normal pixels that are located on a line corresponding to a smallest one of the four difference values.

The videoscope according to the embodiment is **characterized in that** the image processing unit does not correct detection values of the monitoring subject pixels in a still image taking mode and corrects detection values of the monitoring subject pixels if necessary in a moving image taking mode.

The above-described embodiment makes it possible to prevent a flicker on the screen and thereby produce a high-quality image in taking a moving image in a dark shooting environment even if there are pixels each of which produces unstable detection values.

The image correcting method according to the invention is useful when applied to videoscopes because the image correcting method makes it possible to produce a high-quality moving image even in a dark shooting environment even if there are pixels each of which produces unstable detection values.

## Claims

1. A videoscope comprising:
an imaging device comprising: at least one monitoring subject pixel each of which produces, frame by frame, a normal detection value or an abnormal detection value; normal pixels; and at least one defective pixel;
a memory which is stored with a position address of each of the at least one monitoring subject pixel; and
an image processing unit which performs image processing on image signals produced by the imaging device so as to judge, in each frame, based on information stored in the memory, whether each of the at least one monitoring subject pixel has produced a normal detection value or an abnormal detection value and to correct the detection value of each of the at least one monitoring subject pixel using detection values of nearby normal pixels only in frames in which an abnormal detection value is produced.

2. The videoscope according to claim 1, wherein the image processing unit deals with the respective monitoring subject pixel in the same manner as a normal pixel and does not correct a detection value of the monitoring subject pixel if differences between the detection value of the monitoring subject pixel and detection values of nearby normal pixels are within a prescribed range defined by a threshold value.

3. The videoscope according to claim 2, wherein the image processing unit corrects a detection value of the respective monitoring subject pixel if a horizontal difference value between the detection value of the monitoring subject pixel and a detection value or values of a nearby normal pixel or pixels that are located on the same horizontal line as the monitoring subject pixel, a vertical difference value between the detection value of the monitoring subject pixel and a detection value or values of a nearby normal pixel or pixels that are located on the same vertical line as the monitoring subject pixel, a rightward-rising diagonal difference value between the detection value of the monitoring subject pixel and a detection value or values of a nearby normal pixel or pixels that are located on the same rightward-rising diagonal line as the monitoring subj ect pixel, and a leftward-rising diagonal difference value between the detection value of the monitoring subject pixel and a detection value or values of a nearby normal pixel or pixels that are located on the same leftward-rising diagonal line as the monitoring subject pixel are all larger than the threshold value.

4. The videoscope according to claim 3, wherein the image processing unit corrects the detection value of the monitoring subject pixel by replacing it with a detection value of a nearby normal pixel or an average of detection values of nearby normal pixels that are located on a line corresponding to a smallest one of the four difference values.

5. An image correcting method of a videoscope which has an imaging device having at least one monitoring subject pixel each of which produces, frame by frame, a normal detection value or an abnormal detection value, normal pixels and at least one defective pixel, a memory stored with a position address of each of the at least one monitoring subject pixel and an image processing unit, the method comprising:
performing, with the image processing unit, image processing on image signals produced by the imaging device so as to judge, in each frame, based on information stored in the memory, whether each of the at least one monitoring subject pixel has produced a normal detection value or an abnormal detection value and to correct the detection value of each of the at least one monitoring subject pixel using detection values of nearby normal pixels only in frames in which an abnormal detection value is produced.
